(19) Europäisches Patentamt / European Patent Office / Office européen des brevets

(11) EP 2 794 710 B1

(12) EUROPÄISCHE PATENTSCHRIFT

(45) Veröffentlichungstag und Bekanntmachung des Hinweises auf die Patenterteilung:
**15.08.2018 Patentblatt 2018/33**

(21) Anmeldenummer: **12801750.6**

(22) Anmeldetag: **17.12.2012**

(51) Int Cl.:
***C08G 18/42*** (2006.01) ***C08G 18/48*** (2006.01)
***C08G 59/68*** (2006.01) ***C08G 63/82*** (2006.01)
***C08G 18/73*** (2006.01) ***C08G 18/10*** (2006.01)
***A61L 24/04*** (2006.01) ***A61L 24/06*** (2006.01)

(86) Internationale Anmeldenummer:
**PCT/EP2012/075823**

(87) Internationale Veröffentlichungsnummer:
**WO 2013/092504 (27.06.2013 Gazette 2013/26)**

# (54) ISOCYANATFUNKTIONELLES PRÄPOLYMER FÜR EINEN BIOLOGISCH ABBAUBAREN GEWEBEKLEBSTOFF

ISOCYANATE FUNCTIONAL PRE-POLYMER FOR A BIODEGRADABLE TISSUE ADHESIVE

PRÉPOLYMÈRE À ISOCYANATE FONCTIONNEL POUR UN ADHÉSIF TISSULAIRE BIODÉGRADABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 EP 11194416**

(43) Veröffentlichungstag der Anmeldung:
**29.10.2014 Patentblatt 2014/44**

(60) Teilanmeldung:
**18154905.6**

(73) Patentinhaber: **Adhesys Medical GmbH
52074 Aachen (DE)**

(72) Erfinder:
- **HECKROTH, Heike
51519 Odenthal (DE)**
- **EGGERT, Christoph
50733 Köln (DE)**
- **HOFMANN, Jörg
47800 Krefeld (DE)**
- **LORENZ, Klaus
41539 Dormagen (DE)**

(74) Vertreter: **Davepon, Björn
Patentanwaltskanzlei Davepon
Schloss Dyck
41363 Jüchen (DE)**

(56) Entgegenhaltungen:
**EP-A1- 1 752 481**
**EP-A1- 2 336 212**
**WO-A1-2006/010278**
**WO-A1-2011/047789**
**US-A1- 2003 032 734**
**US-A1- 2005 129 733**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 794 710 B1

**Beschreibung**

Die vorliegende Erfindung betrifft ein isocyanatfunktionelles Präpolymer sowie ein Verfahren zu dessen Herstellung. Die Erfindung betrifft außerdem ein Gewebekleber-System, das ein solches isocyanatfunktionelles Präpolymer enthält und ein Dosiersystem mit wenigstens zwei Kammern und einem derartigen Gewebeklebersystem.

In der jüngeren Vergangenheit hat sich ein steigendes Interesse entwickelt, chirurgische Nähte durch den Einsatz von geeigneten Klebstoffen zu ersetzen oder zu unterstützen. Besonders im Bereich der plastischen Chirurgie, in der auf dünne, möglichst unsichtbare Narben Wert gelegt wird, werden vermehrt Klebstoffe eingesetzt.

Um bei Chirurgen als Nahtersatz akzeptiert zu werden, müssen Gewebeklebstoffe eine Reihe von besonderen Eigenschaften besitzen. Hierzu gehören eine leichte Verarbeitbarkeit und eine ausreichende Viskosität, so dass der Klebstoff nicht in tiefere Gewebeschichten eindringen oder verlaufen kann. In der klassischen Chirurgie wird zusätzlich eine schnelle Aushärtung gefordert, wogegen in der plastischen Chirurgie eine Korrektur der Klebenaht möglich sein soll und damit die Aushärtungsgeschwindigkeit nicht zu hoch sein darf. Der Klebstoff muss darüber hinaus biokompatibel sein und darf weder Histotoxicität noch Thrombogenität oder ein allergenes Potential besitzen.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Die schnelle Aushärtezeit, sowie die Sprödigkeit der Klebestelle limitieren jedoch den Einsatz. Durch die schlechte Bioabbaubarkeit sind Cyanacrylate nur für äußere Anwendung geeignet.

Als Alternative zu den Cyanacrylaten stehen biologische Klebstoffe wie zum Beispiel BioGlue®, eine Mischung aus Glutaraldehyd und Bovinem Serumalbumin, diverse kollagen- und gelatinebasierte Systeme (FloSeal®) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal® dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsätzen etc. nicht möglich ist. Hier steht zur Behandlung traumatischer Wunden QuikClot® oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der EP 2 011 808 A1 sind Gewebekleber bekannt, die auf einem hydrophilen 2-Komponenten-Polyurethan-System beruhen. Diese Gewebekleber können zum Abdecken, Verschließen oder Verkleben von Zellgewebe und insbesondere zum Verkleben von Wunden eingesetzt werden. Die beschriebenen Gewebekleber zeichnen sich dabei durch eine starke Bindung zum Gewebe, eine hohe Flexibilität der erhaltenen Fügenaht, eine leichte Applizierbarkeit, eine in einem weiten Bereich einstellbare Aushärtungszeit und eine hohe Biokompatibilität aus.

Allerdings treten bei der Verwendung der bekannten Gewebekleber auch bestimmte Probleme auf. So kann es aufgrund der Hydrophilie der Polyurethan-Systeme bei längerer Exposition mit Wasser zu einer Quellung des Gewebeklebers kommen. Hierdurch wird die Haftung des Gewebeklebers zum Gewebe verringert, was sich insgesamt negativ auf die Haltbarkeit der Verklebung auswirken kann.

Aus der EP 2 145 634 A1 sind 2K-Klebstoff-Systeme auf Basis von Polyharnstoff bekannt. Diese sind durch Umsetzung von isocyanatfunktionellen Präpolymeren auf Basis aliphatischer Isocyanate mit speziellen, strukturell von Aminosäuren abgeleiteten, sekundären Diaminen erhältlich. Diese Klebstoff-Systeme eignen sich besonders als Hämostatikum zur Blutstillung und weisen daneben vorteilhafte Klebeigenschaft auf. Ferner können mit diesen Systemen insbesondere auch bei größeren Verletzungen Gewebestücke wieder aneinandergefügt bzw. miteinander verbunden werden, was für den Wundheilungsprozess vorteilhaft ist.

Der aus der EP 2 145 634 A1 bekannte Polyharnstoff-basierte Klebstoff ist jedoch im Wesentlichen für eine externe Anwendung ausgelegt. So ist es für eine Anwendung im Körper notwendig, dass sich der Klebstoff dort nach Beendigung der Wundheilung zeitnah abbaut. Dies ist bei dem bekannten Klebstoff nicht der Fall.

Aus der WO 2009/106245 A2 ist die Herstellung und Verwendung von Polyharnstoff-Systemen als Gewebekleber bekannt. Die hier offenbarten Systeme umfassen wenigstens zwei Komponenten. Dabei handelt es sich um einen aminofunktionellen Asparaginsäureester und ein isocyanatfunktionelles Präpolymer, das durch Umsetzung von aliphatischen Polyisocyanaten mit Polyesterpolyolen erhältlich ist. Die beschriebenen 2-Komponenten Polyharnstoff-Systeme können als Gewebekleber für den Verschluss von Wunden in menschlichen und tierischen Zellverbänden eingesetzt werden. Dabei kann ein sehr gutes Klebeergebnis erzielt werden. Die Polyharnstoff-Systeme sind dafür ausgelegt, innerhalb einer Zeit von bis zu 6 Monaten biologisch abgebaut zu werden.

Bei den aus der WO 2009/106245 A2 bekannten Systemen befindet sich die primär beim biologischen Abbau gespaltene Estergruppe in der Polyolkomponente des verwendeten Präpolymers. Die Herstellung einer entsprechenden

Komponente ist mit einem relativ großen Aufwand verbunden.

Aus der EP 2 336 212 A1 ist ein Klebstoffverbundsystem bekannt, das aus einem Gewebekmeber, der auf hydrophilen Polyurethanpolymeren basiert, und einer wasserundurchlässigen Schutzschicht besteht. Die Schutzschicht soll ein Aufquellen des Gewebeklebers verhindern.

Aus der US 2003/032734 A1 sind 1- und 2-Komponenten-Gewebekleber bekannt. Die 1-Komponenten-Gewebekeber bestehen nur aus einem Polyethylenoxid mit zwei oder mehr Isocyanat-Substituent, während die 2-Komponenten-Gewebekleber zusätzlich ein bioabsorbierbares Diamin enthalten.

Aus der US 2005/0129733 A1 ist ein flüssiger Gewebekleber bekannte, der zu einem Feststoff aushärtet und zur dauerhaften Befestigung von Gewebe im Körper verwendet werden kann, Der Gewebekleber enthält einen Polyether-Polyol mit endständigen Isocyanatgruppcn und ein Polyisocyanat.

Es war daher wünschenswert, ein neues Präpolymer bereit zu stellen, das einerseits leichter zugänglich ist und andererseits eine zusätzliche oder alternative, unter physiologischen Bedingungen spaltbare funktionelle Gruppe aufweist.

Aufgabe der Erfindung war es daher, ein Präpolymer zur Verfügung zu stellen, das leicht zugänglich ist, eine gute Abbaubarkeit unter physiologischen Bedingungen aufweist und unter physiologischen Bedingungen mit einem Härter schnell zu einem Material mit guten Klebeeigenschaften ausreagiert.

Diese Aufgabe wurde gelöst durch ein Gewebekleber-System, umfassend einen Gewebekleber mit einer Komponente A) sowie einer Komponente B) und/ oder einer Komponente C), wobei
die Komponente A) ein isucyanatfunktionelles Präpolymer ist, erhältlich durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppentragenden Vorstufe, wobei das Co-Monomer ausgewählt ist aus der Gruppe umfassend Lactide, Glycolide, sowie Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie

b) Umsetzung der Hydroxylgruppen tragenden Vorstufe aus Schritt a) mit einem polyfunktionellen Isocyanat zu dem isocyanatfunktionellen Präpolymer,

wobei die Komponente B) ein aminofunktioneller Asparaginsäureester der allgemeinen Formel (I)

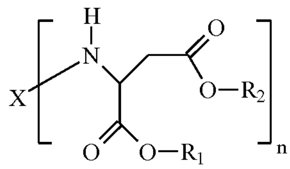

(I)

ist,
wobei

X ein n-wertiger organischer Rest ist,

Rl1, R2 gleiche oder verschiedene organische Reste sind, die keine Zerewitinoff-aktiven H-Atome aufweisen,

n eine ganze Zahl ≥ 2 ist, wobei der aminofunktionelle Asparaginsäureester ein solcher der allgemeinen Formel (VIII)

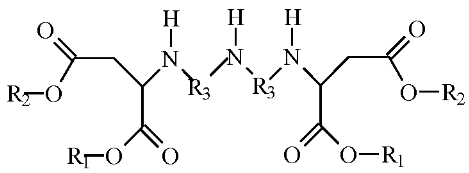

(VIII)

ist,

wobei $R_1$, $R_2$, $R_3$ gleiche oder verschiedene organische Reste sind, die keine Zerewitinoff-aktiven H-Atome aufweisen,

und wobei die Komponente C) ein Umsetzungsprodukt des isocyanatfunktionellen Präpolymers A) mit dem aminofunktionellen Asparaginsäureester B) ist.

Offenbart ist ferner ein isocyanatfunktionelles Präpolymer, das erhältlich ist durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, cyclische Dicarbonsäureanhydride sowie Kombinationen hiervon und wobei das Co-Monomer durch eine statistische Copolymerisation m die Polymerkette(n) der Hydroxylgruppen tragenden Vorstufe eingebaut ist, sowie

b) Umsetzung der Hydroxylgruppen tragenden Vorstufe aus Schritt a) mit einem polyfunktionellen Isocyanat zu einem isocyanatfunktionellen Präpolymer.

Mit anderen Worten enthält das erfindungsgemäße isocyanatfunktionelle Präpolymer in den Polymerketten Estergruppen, die durch statistische Copolymerisation von Alkylenoxidverbindungen und Lactiden, Glycoliden und / oder cyclischen Dicarbonsäureanhydriden auf Zerewitinoff-aktive H-Atome enthaltende Starterverbindungen erzeugt werden. Insbesondere sind die Estergruppen nicht blockweise eingebaut. Dabei ist unter dem unbestimmten Artikel "ein", "einer" usw. zu verstehen, dass wahlweise jeweils auch mehrere dieser Komponenten miteinander umgesetzt werden können. Die genannten Komponenten, insbesondere das Co-Monomer, kann auch dimer, trimer usw. eingesetzt werden, wie beispielsweise als Dilactid.

Überraschenderweise hat sich gezeigt, dass solche isocyanatfunktionellen Präpolymere beispielsweise im Körper eines Patienten biologisch abbaubar sind. Die Abbauzeit liegt dabei oberhalb der Heilungsdauer für die zu verschließende Wunde, beispielsweise bei 4 Wochen. Hierbei scheint sich insbesondere die statistische Verteilung der Co-Monomereinheiten in der (den) Polymerkette(n) vorteilhaft auf die Abbaugeschwindigkeit auszuwirken, da diese als "Sollbruchstellen" in dem ausgehärteten Klebstoff fungieren. Wenn die Co-Monomerbausteine beim biologischen Abbau angegriffen und aufgebrochen werden, verkürzt sich dadurch die Polymerkettenlänge besonders schnell.

Gleichzeitig zeichnen sich die erfindungsgemäßen isocyanatfunktionellen Präpolymere durch eine hohe Adhäsion insbesondere auf menschlichem oder tierischem Gewebe sowie eine hohe Aushärtungsgeschwindigkeit aus. Zudem erfüllen Gewebekleber-Systeme mit einem erfindungsgemäßen isocyanatfunktionellen Präpolymer die Anforderungen hinsichtlich der oben angesprochenen Histotoxizität, Thrombogenität und allergenes Potential.

Im Rahmen der vorliegenden Erfindung ist vorgesehen, dass die H-funktionelle Starterverbindung wenigstens ein Zerewitinoff-aktives H-Atom trägt. Unter einem Zerewitinoff-aktiven H-Atom wird im Rahmen der vorliegenden Erfindung ein azides H-Atom oder "aktives" H-Atom verstanden. Ein solches kann in an sich bekannter Weise durch eine Reaktion mit einem entsprechenden Grignard-Reagenz ermittelt werden. Die Menge an Zerewitinoff-aktiven H-Atomen wird typischerweise über die Methanfreisetzung gemessen, die bei einer Reaktion der zu überprüfenden Substanz mit Methylmagnesiumbromid (CH3-MgBr) gemäß der folgenden Reaktionsgleichung frei wird:

$$CH_3\text{-}MgBr + ROH \rightarrow CH_4 + Mg\,(OR)Br$$

Zerewitinoff-aktive H-Atome stammen typischerweise von C-H aziden organischen Gruppen, -OH, -SH, -NH2 oder -NHR mit R als organischem Rest sowie -COOH.

Besonders geeignete H-funktionelle Starterverbindungen besitzen eine H-Funktionalität von 1 bis 35, insbesondere 1 bis 16, bevorzugt 1 bis 8, wobei sich die H-Funktionalität auf die vorgenannten Zerewitinoff-aktiven H-Atome bezieht.

Als H-funktionelle Starterverbindungen sind insbesondere polyhydroxyfunktionelle Polymere geeignet, welche insbesondere ausgewählt sind aus geradkettigen und/oder verzweigten Polyethern, Polyestern, Polyetherpolyestern, Polycarbonaten, Polyetherpolycarbonaten, sowie Kombinationen hiervon.

Sofern das als H-funktionelle Starterverbindung eingesetzte Hydroxylgruppen tragende Polymer ein Polyether ist oder Polyethergruppen aufweist, enthalten diese weiter bevorzugt Ethylenoxideinheiten, wobei der Gewichtsanteil der Ethylenoxideinheiten in einem solchen vorgefertigten Alkylenoxidadditionsprodukt insbesondere wenigstens 40 Gew.% beträgt, bevorzugt wenigstens 50 Gew.%. Beispielsweise beträgt der Gewichtsanteil an Ethylenoxideinheiten 40 bis 90 Gew.-%, bevorzugt 50 bis 80 Gew.-%, jeweils bezogen auf die Masse des Hydroxylgruppen tragenden Polymers. Der Rest des Polyethergerüsts bzw. der Polyetherbausteine kann jeweils durch andere Alkylenoxideinheiten wie

insbesondere (Poly)propylenoxid, (Poly)butylenoxid oder andere (Poly)Alkylenoxidgruppen und Mischungen hiervon aufgebaut sein. Die Molekulargewichte der H-funktionellen Starterverbindung können über weite Bereiche variieren. So kann das mittlere Molgewicht beispielsweise 17 bis 10000 g/mol betragen, insbesondere von mehr als 200 bis 9000 g/mol. Das mittlere Molgewicht bezeichnet bei polymeren Verbindungen deren Zahlenmittel, welches über an sich bekannte Methoden, beispielsweise über Gelpermeationschromatographie oder die Ermittlung der OH-Zahl bestimmbar ist. Mit anderen Worten kann als H-funktionelle Starterverbindung für das erfindungsgemäße Präpolymer eine monomere Starterverbindung gewählt werden, wie beispielsweise Ammoniak oder Ethylenglykol. Auch oligomere Starterverbindungen sind umfasst, beispielsweise Polyether mit einem mittleren Molgewicht von 200 bis 600 g/mol sowie polymere Starterverbindungen mit höheren Molekulargewichten, beispielsweise von mehr als 600 bis 10000 g/mol oder 800 bis 9000 g/mol.

Neben den bevorzugt zu verwendenden hydroxyfunktionellen Startern können auch aminofunktionelle Starter eingesetzt werden. Beispiele für hydroxyfunktionelle Starterverbindungen sind Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, sowie methylolgruppenhaltige Kondensate aus Formaldehyd und Phenol oder Harnstoff. Es können auch hochfunktionelle Starterverbindungen auf Basis von hydrierten Stärkehydrolyseprodukten eingesetzt werden. Solche sind beispielsweise in EP 1525244 A1 beschrieben.

Beispiele für aminogruppenhaltige H-funktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans sowie bei der Kondensation von Anilin mit Formaldehyd zu Diaminodiphenylmethan anfallende höherkernige Produkte, ferner methylolgruppenhaltige Kondensate aus Formaldehyd und Melamin sowie Mannichbasen. Außerdem können als Starterverbindungen auch Ringöffnungsprodukte aus cyclischen Carbonsäureanhydriden und Polyolen eingesetzt werden. Beispiele sind Ringöffnungsprodukte aus Phthalsäureanhydrid oder Bernsteinsäureanhydrid einerseits und Ethylenglykol, Diethylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit oder Sorbit andererseits. Daneben ist es auch möglich, ein- oder mehrfunktionelle Carbonsäuren direkt als Starterverbindungen einzusetzen.

Ferner können in dem Prozess auch vorgefertigte Alkylenoxidadditionsprodukte der erwähnten Starterverbindungen, also Polyetherpolyole vorzugsweise mit OH-Zahlen von 5 bis 1000 mg KOH/g, bevorzugt 10 bis 1000 mg KOH/g, als Starterverbindungen eingesetzt, bzw. dem Reaktionsgemisch zugesetzt werden. Auch ist es möglich, im erfindungsgemäßen Prozess Polyesterpolyole vorzugsweise mit OH-Zahlen im Bereich von 6 bis 800 mg KOH/g als Starter oder Co-Starter einzusetzen. Hierfür geeignete Polyesterpolyole können beispielsweise aus organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden.

Des Weiteren können als H-funktionelle Startersubstanzen Polycarbonatpolyole, Polyestercarbonatpolyole oder Polyethercarbonatpolyole, bevorzugt Polycarbonatdiole, Polyestercarbonatdiole oder Polyethercarbonatdiole vorzugsweise jeweils mit OH-Zahlen im Bereich von 6 bis 800 mg KOH/g, als Starter oder Co-Starter verwendet werden. Diese werden beispielsweise durch Umsetzung von Phosgen, Dimethylcarbonat, Diethylcarbonat oder Diphenylcarbonat mit di- oder höherfunktionellen Alkoholen oder Polyesterpolyolen oder Polyetherpolyolen hergestellt.

Eingesetzt werden können auch Polyethercarbonatpolyole, wie sie beispielsweise durch katalytische Umsetzung von Alkylenoxiden (Epoxiden) und Kohlendioxid in Anwesenheit von H-funktionellen Startersubstanzen erhältlich sind (siehe z.B. EP-A 2046861). Diese Polyethercarbonatpolyole haben bevorzugt eine OH-Zahl von $\geq$ 5 mg KOH / g bis $\leq$ 240 mg KOH/g, besonders bevorzugt von $\geq$ 9 bis $\leq$ 200 mg KOH/g.

In Schritt a) der Herstellung des erfindungsgemäßen Präpolymers dienen bevorzugt aminogruppenfreie H-funktionelle Starterverbindungen mit Hydroxylgruppen als Träger der aktiven Wasserstoffe wie beispielsweise Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol und hydrierte Stärkehydrolyseprodukte. Es können auch Gemische verschiedener H-funktioneller Starterverbindungen eingesetzt werden.

Als erfindungsgemäß verwendbare Alkylenoxidverbindungen können solche Vertreter ausgewählt sein, die 2 bis 24 Kohlenstoffatome aufweisen, insbesondere 2 bis 12 Kohlenstoffatome, weiter bevorzugt 2 bis 6 Kohlenstoffatome, sowie die Kombination unterschiedlicher Alkylenoxidverbindungen der vorgenannten Art. Bei den Epoxiden mit 2 bis 24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid,

2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, C1-C24-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxypropyltrimethoxysilan, 3-Glycidyloxypropyltriethoxysilan, 3-Glycidyloxypropyltripropoxysilan, 3 -Glycidyloxypropyl-methyldimethoxysilan, 3-Glycidyloxypropyl-ethyldiethoxysilan und 3-Glycidyloxypropyltriisopropoxysilan. Vorzugsweise werden Ethylenoxid und/oder Propylenoxid verwendet. Insbesondere kann der Gewichtsanteil an Ethylenoxid, bezogen auf die Gesamtmasse der dosierten Alkylenoxidverbindungen, wenigstens 40 Gew.-% betragen, bevorzugt wenigstens 50 Gew.-%. Beispielsweise beträgt der Gewichtsanteil an Ethylenoxid 40 - 90 %, bevorzugt 50 - 80 %, jeweils bezogen auf die Gesamtmasse der dosierten Alkylenoxidverbindungen.

Es ist erfindungsgemäß vorgesehen, dass das isocyanatfunktionelle Präpolymer Bausteine herrührend von Lactiden, Glycoliden und / oder cyclischen Dicarbonsäureanhydriden enthält, die durch eine statistische Copolymerisation in die Polymerkette der Hydroxylgruppen tragenden Vorstufe eingebaut sind. In bevorzugter Weise beträgt das Stoffmengenverhältnis der Alkylenoxidverbindung zu diesem Co-Monomer in der Hydroxylgruppen tragenden Vorstufe 200 : 1 bis 1 : 1 beträgt, insbesondere 10 : 1 bis 5 : 1. Diese Stoffmengenverhältnisse sind besonders bevorzugt, weil ein Gewebekleber enthaltend eine solche Präpolymervorstufe ein gutes Adhäsionsvermögen bei geringer Aushärtezeit besitzt und sich zudem unter physiologischen Bedingungen schnell abbaut.

Grundsätzlich ist es auch möglich, weitere Co-Monomere, wie z.B. cylische Anhydride oder Kohlendioxid durch statistische Copolymerisation in die Polymerkette des Hydroxylgruppen tragenden Präpolymers mit einzubauen.

In bevorzugter Weise kann vorgesehen sein, dass das in Schritt b) eingesetzte polyfunktionelle Isocyanat ausgewählt ist aus aliphatischen Isocyanaten, insbesondere aus Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat, Diisocyanatododecan oder Kombinationen hiervon. Hierbei sind Hexamethylendiisocyanat (HDI), Isophorondiisocyanat (IPDI), Butylendiisocyanat (BDI) und Bis(isocyanatocyclohexyl)methan (HMDI) bevorzugt. Besonders bevorzugt sind Hexamethylendiisocyanat, Isophorondiisocyanat, Butylendiisocyanat, ganz besonders bevorzugt Hexamethylendiisocyanat und Isophorondiisocyanat.

Grundsätzlich ist die Erfindung jedoch nicht auf den Einsatz aliphatischer Isocyanate beschränkt, es können also auch übliche aromatische Isocyanate verwendet werden, wie beispielsweise Toluol-diisocyanat (TDI) oder Diphenylmethandiisocyanat (MDI).

Die Umsetzung des nach Schritt a) erhaltenen Hydroxylgruppen tragenden Präpolymers mit dem polyfunktionellen Isocyanat in Schritt b) kann bei einem NCO/OH-Verhältnis von 4:1 bis 12:1 erfolgen, bevorzugt 8:1, und anschließend kann der Anteil an nicht umgesetztem Isocyanat mittels geeigneter Methoden abgetrennt werden. Üblicherweise wird hierfür die Dünnschichtdestillation verwendet, wobei ein Präpolymer mit einem Restmonomergehalt von weniger als 1 Gew.-%, bevorzugt weniger als 0,1 Gew.-%, ganz besonders bevorzugt weniger als 0,03 Gew.-% erhalten wird.

Gegebenenfalls können während der Herstellung des isocyanatfunktionellen Präpolymers Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Umsetzung im Schritt b) beträgt bevorzugt 20 bis 120 °C und weiter bevorzugt 60 bis 100 °C.

Das isocyanatfunktionelle Präpolymer hat bevorzugt einen nach DIN EN ISO 11909 gemessenen mittleren NCO-Gehalt von 2 bis 10 Gew.-%, bevorzugt von 2,5 bis 8 Gew.-%.

Die mittlere NCO-Funktionalität des isocyanatfunktionellen Präpolymers beträgt bevorzugt 1,5 bis 6, weiter bevorzugt 1,6 bis 5, noch weiter bevorzugt 1,7 bis 4, ganz besonders bevorzugt 1,8 bis 3,5 und insbesondere 3.

Ein weiterer Gegenstand der vorliegenden Erfindung betrifft ein Verfahren zur Herstellung eines isocyanatfunktionellen Präpolymers, umfassend die Schritte:

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, cyclische Dicarbonsäureanhydride sowie Kombinationen hiervon und wobei das Co-Monomer durch eine statistische Copolymerisation in die Polymerkette(n) der Hydroxylgruppen tragenden Vorstufe eingebaut ist, sowie

b) Umsetzung der Hydroxylgruppen tragenden Vorstufe aus Schritt a) mit einem polyfunktionellen Isocyanat zu einem isocyanatfunktionellen Präpolymer.

Das erfindungsgemäße Verfahren kann unkatalysiert durchgeführt werden, wobei der Einsatz eines Katalysators jedoch bevorzugt ist. Hierbei kann insbesondere der Schritt a) über einen Doppelmetallcyanid-Katalysator (DMC-Katalysator) katalysiert werden, der insbesondere Zinkhexacyanocobaltat (III), Zinkhexacyanoiridat (III), Zinkhexacyanoferrat (III) oder Cobalt(II) hexacyanocobaltat (III) enthält.

Ein besonderer Vorteil dieser Ausgestaltung des erfindungsgemäßen Verfahrens liegt darin, dass die als Zwischenprodukt des Schrittes a) erhaltene Hydroxylgruppen tragende Vorstufe eine vergleichbar enge Molekülkettenlängen-Verteilung aufweist. Einer der Gründe kann in dem Einsatz der DMC-Katalyse gesehen werden, denn solche Katalysatoren zeigen eine sogenannte "catch-up"-Kinetik. Das heißt, dass mit zunehmender Kettenlänge die katalytische Aktivität für die Anbindung des nächsten Monomerbausteins sukzessive abnimmt und damit auch die Reaktionsgeschwindigkeit.

Dieser Effekt kann auch dazu verwendet werden, das erfindungsgemäße Verfahren halb- oder auch vollkontinuierlich zu betreiben. Bei halbkontinuierlicher Verfahrensweise wird eine bestimmte Menge einer Hydroxylgruppen tragenden Vorstufe als Lösungsmittel vorgelegt, wobei diese Hydroxylgruppen tragende Vorstufe aus einem vorangehenden Herstellungsdurchlauf von Schritt a) des erfindungsgemäßen Verfahrens stammt oder auch aus anderen Quellen stammen kann. Der DMC-Katalysator sorgt nun dafür, dass die neu zu synthetisierende Hydroxylgruppen-tragende Vorstufe so weit aufgebaut wird, bis diese in etwa die Kettenlänge der als "Lösungsmittel" verwendeten Hydroxylgruppen-tragenden Vorstufe früherer Herstellung erreicht. Der Reaktionsmischung kann dann ein bestimmter Produktanteil an Hydroxylgruppen-tragender Vorstufe entnommen werden, beispielsweise 90%, um damit Schritt b) durchzuführen, wobei die verbleibenden 10% als Lösungsmittel für den erneuten Reaktionsdurchlauf von Schritt a) zurückbleiben.

Geeignete DMC-Katalysatoren sind im Prinzip aus dem Stand der Technik bekannt und werden beispielsweise in US 3,404,109 A1, US 3,829,505 A1, US 3,941,849 A1 und US 5,158,922 A1 offenbart.

DMC-Katalysatoren, die z.B. in US 5,470,813 A1, EP 700949 A1, EP 743 093 A1, EP 761 708 A1, WO 97/40086 A1, WO 98/16310 A1 und WO 00/47649 A1 beschrieben sind, besitzen eine sehr hohe Aktivität in der Polymerisation von Alkylenoxiden und ermöglichen die Herstellung von Polyetherpolyolen bei sehr geringen Katalysatorkonzentrationen (25 ppm oder weniger), so dass eine Abtrennung des Katalysators aus dem fertigen Produkt im Allgemeinen nicht mehr erforderlich ist. Ein typisches Beispiel sind die in EP 700 949 A1 beschriebenen hochaktiven DMC-Katalysatoren, die neben einer Doppelmetallcyanid-Verbindung wie Zinkhexacyanocobaltat(III) und einem organischen Komplexliganden wie tert.-Butanol noch einen Polyether mit einem zahlenmittlerem Molekulargewicht größer als 500 g/mol enthalten. Es ist auch möglich, die in EP Anmeldenummer 10163170.3 offenbarten alkalischen DMC-Katalysatoren einzusetzen.

Zur Herstellung der Doppelmetallcyanid-Verbindungen geeignete cyanidfreie Metallsalze besitzen bevorzugt die allgemeine Formel (II),

$$M(X)_n \qquad (II)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$,

X für ein oder mehrere (d.h.verschiedene) Anionen steht, welches vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

n ist 1, wenn X = Sulfat, Carbonat oder Oxalat ist und

n ist 2, wenn X = Halogenid, Hydroxid, Cyanat, Thiocyanat, Isocyanat, Isothiocyanat oder Nitrat ist.

Weiter geeignete cyanidfreie Metallsalze besitzen die allgemeine Formel (III),

$$M_r(X)_3 \qquad (III)$$

wobei

M ausgewählt ist aus den Metallkationen $Fe^{3+}$, $Al^{3+}$ und $Cr^{3+}$,

X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

r ist 2, wenn X = Sulfat, Carbonat oder Oxalate ist und

r ist 1, wenn X = Halogenid, Hydroxid, Cyanat, Thiocyanat, Isocyanat, Isothiocyanat, Carboxylat oder Nitrat ist.

Andere geeignete cyanidfreie Metallsalze besitzen die allgemeine Formel (IV),

$$M(X)_s \qquad (IV)$$

wobei

M ausgewählt ist aus den Metallkationen $Mo^{4+}$, $V^{4+}$ und $W^{4+}$

X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

s ist 2, wenn X = Sulfat, Carbonat oder Oxalat ist und

s ist 4, wenn X = Halogenid, Hydroxid, Cyanat, Thiocyanat, Isocyanat, Isothiocyanat, Carboxylat oder Nitrat ist.

Ebenfalls geeignete cyanidfreie Metallsalze besitzen die allgemeine Formel (V),

$$M(X)_t \qquad (V)$$

wobei

M ausgewählt ist aus den Metallkationen $Mo^{6+}$ und $W^{6+}$

X steht für einen oder unterschiedliche Anionentypen, wobei das Anion vorzugsweise ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

t ist 3, wenn X = Sulfat, Carbonat oder Oxalat ist und

t ist 6, wenn X = Halogenid, Hydroxid, Cyanat, Thiocyanat, Isocyanat, Isothiocyanat, Carboxylat oder Nitrat ist,

Beispiele geeigneter cyanidfreier Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel-(II)nitrat. Es können auch Mischungen verschiedener Metallsalze eingesetzt werden.

Zur Herstellung der Doppelmetallcyanid-Verbindungen geeignete Metallcyanidsalze besitzen bevorzugt die allgemeine Formel (VI)

$$(Y)_aM'(CN)_b(A)_c \qquad (VI)$$

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),

Y ausgewählt ist aus einem oder mehreren Metallkationen der Gruppe bestehend aus Alkalimetall (d.h. $Li^+$, $Na^+$, $K^+$, $Rb^+$, $Cs^+$) und Erdalkalimetall (d.h. $Be^{2+}$, $Ca^{2+}$, $Mg^{2+}$, $Sr^{2+}$, $Ba^{2+}$),

A ausgewählt ist aus einem oder mehreren Anionen der Gruppe bestehend aus Halogeniden (d..h. Fluorid, Chlorid, Bromid, Iodid), Hydroxid, Sulfat, Carbonat, Cyanat, Thiocyanat, Isocyanat, Isothiocyanat, Carboxylat, Oxalat oder Nitrat und

a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).

Bevorzugte Doppelmetallcyanid-Verbindungen, die in den erfindungsgemäßen DMC-Katalysatoren enthalten sind, sind Verbindungen der allgemeinen Formel (VII)

$$M_X[M'_{X'}(CN)_Y]_Z \qquad (VII),$$

worin M wie in Formel (II) bis (IV),

und M' wie in Formel (VI) definiert ist, und

x, x', y und z sind ganzzahlig und so gewählt, dass die Elektronenneutralität der Doppel- metallcyanidverbindung gegeben ist.

Vorzugsweise ist

x = 3, x' = 1,y = 6 und z = 2,

M = Zn(II), Fe(II), Co(II) oderNi(II) und

M' = Co(III), Fe(III), Cr(III) oder Ir(III).

Beispiele bevorzugt eingesetzter Doppelmetallcyanidverbindungen sind Zinkhexacyanocobaltat(III), Zinkhexacyanoiridat(III), Zinkhexacyanoferrat(III) und Cobalt(II)hexacyanocobaltat(III). Weitere Beispiele geeigneter Doppelmetallcyanid-Verbindungen sind z.B. US 5,158,922 A1 zu entnehmen. Besonders bevorzugt verwendet wird Zinkhexacyanocobaltat(III).

Die bei der Herstellung der DMC-Katalysatoren zugesetzten organischen Komplexliganden sind beispielsweise in US-A 5,158,922 A1, US 3,404,109 A1, US 3,829,505 A1, US 3,941,849 A1, EP 700949 A1, EP 761708 A1, JP 4145123 A1, US 5,470,813 A1, EP 743 093 A1 und WO 97/40086 A1 offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid - Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der erfindungsgemäß bevorzugten DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der erfindungsgemäß bevorzugten DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Metallcyanidsalz, (also mindestens ein molares Verhältnis von cyanidfreiem Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält. Der organische Komplexligand kann dabei in der wässrigen Lösung des cyanidfreien Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Metallcyanidsalzes und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d.h. der Herstellung der Suspension) erfolgt unter Einsatz einer Mischdüse, besonders bevorzugt unter Einsatz eines Strahldispergators wie in WO 01/39883 A1 beschrieben.

Im zweiten Schritt erfolgt die Isolierung des Feststoffs (d.h. die Vorstufe des erfindungsgemäßen Katalysators) aus der Suspension durch bekannte Techniken, wie Zentrifugation oder Filtration.

In einer bevorzugten Ausführungsvariante zur Herstellung des Katalysators wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem erfindungsgemäßen Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung eine weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen. Hierzu kann z.B. der erste Waschvorgang wiederholt werden. Bevorzugt ist es aber, für weitere Waschvorgänge nicht wässrige Lösungen zu verwenden, z.B. eine Mischung aus organischem Komplexliganden und weiterer komplexbildender Komponente.

Der isolierte und gegebenenfalls gewaschene Feststoff wird anschließend, gegebenenfalls nach Pulverisierung, bei Temperaturen von im allgemeinen 20 - 100 °C und bei Drücken von im allgemeinen 0,1 mbar bis Normaldruck (1013 mbar) getrocknet.

Ein bevorzugtes Verfahren zur Isolierung der erfindungsgemäßen DMC-Katalysatoren aus der Suspension durch Filtration, Filterkuchenwäsche und Trocknung wird in WO 01/80994 A1 beschrieben.

Die Konzentration an in Schritt a) eingesetztem DMC-Katalysator beträgt 5 bis 1000 ppm, bevorzugt 10 bis 900 ppm und besonders bevorzugt 20 bis 500 ppm, bezogen auf die Menge des herzustellenden Hydroxylgruppen tragenden Präpolymers. Je nach Anforderungsprofil der Anwendung kann der DMC-Katalysator im Produkt belassen oder (teilweise) abgetrennt werden Die (teilweise) Abtrennung des DMC-Katalysators kann beispielsweise durch Behandlung mit Adsorbentien erfolgen. Verfahren zur Abtrennung von DMC-Katalysatoren sind beispielsweise beschrieben in US 4,987,271 A1, DE 313 22 58 A1, EP 406 440 A1, US 5,391,722 A1, US 5,099,075 A1, US 4,721,818 A1, US 4,877,906 A1 und EP 385 619 A1.

Wird das erfindungsgemäße Verfahren unter Verwendung von Doppelmetallcyanid-Katalysatoren durchgeführt, so ist es weiterhin von Vorteil, zunächst die H-funktionelle Starterverbindung, den Katalysator und das Co-Monomer vorzulegen und anschließend die Alkylenoxidverbindung zuzudosieren. Diese Vorgehensweise wirkt sich vorteilhaft auf die statistische Verteilung der Co-Monomerbausteine in der Polymerkette aus und verbessert dadurch die biologische Abbaubarkeit des Gewebekleber-Systems.

Im Folgenden wird Schritt a) des erfindungsgemäßen Verfahrens detailliert beschrieben, wobei die vorliegende Erfindung nicht auf die folgende Darstellung beschränkt ist:

In einer Ausführungsform des erfindungsgemäßen Verfahrens wird die H-funktionelle Verbindung mit dem DMC-Katalysator und dem Co-Monomer zunächst in einem Reaktor / Reaktorsystem vorgelegt. Dabei kann insbesondere bereits die gesamte Co-Monomermenge zu Beginn vorgelegt werden. Der H-funktionellen Verbindung können ggf. vor Inkontaktbringen mit dem DMC-Katalysator geringe Mengen einer anorganischen Mineralsäure, bevorzugt Phosphorsäure, zugesetzt werden, um etwaige Basenspuren in der H-funktionellen Starterverbindung zu neutralisieren, bzw. um den Produktionsprozess generell stabiler zu gestalten.

Alternativ hierzu kann auch nur die H-funktionelle Verbindung mit dem DMC-Katalysator vorgelegt werden und anschließend das Co-Monomer sowie das Alkylenoxid kontinuierlich zugeführt werden, insbesondere parallel.

Nach einer weiteren Verfahrensvariante wird zunächst die H-funktionelle Verbindung sowie der DMC-Katalysator vorgelegt und anschließend eine Teilmenge des Alkylenoxids zudosiert. Dadurch kann die Kettenlänge der H-funktionellen Verbindung zunächst unter Bildung von Oxyalkyleneinheiten vergrößert werden, was sich insbesondere bei niedermolekularen H-funktionellen Verbindungen anbietet, wie beispielsweise bei solchen mit einem mittleren Molgewicht von bis zu 600 g/mol. In diesem Abschnitt sind dann noch keine Bausteine des Co-Monomeren vorhanden. Anschließend kann dann das Co-Monomer sowie weiteres bzw. das restliche Alkylenoxid zudosiert werden, um die Co-Polymerisation durchzuführen. Hierbei kann entweder zunächst die gesamte Menge an Co-Monomer zugeführt und das restliche Alkylenoxid anschließend eingeleitet werden. Auch ist es möglich, das Co-Monomer sowie das restliche Alkylenoxid gleichzeitig kontinuierlich zuzuführen.

Zudem sind auch andere Mischformen zwischen den vorgenannten Verfahrensvarianten denkbar.

Nach der Vorstellung dieser grundsätzlichen Varianten sollen die Einzelheiten der Verfahrensdurchführung näher erläutert werden. Nach Aufheizen auf Temperaturen von 50 bis 160 °C, insbesondere 60 bis 140 °C, ganz besonders bevorzugt 70 bis 140 °C wird der Reaktorinhalt in einer bevorzugten Verfahrensvariante vor Beginn der Alkylenoxiddosierung mit Inertgas über einen Zeitraum bevorzugt 10 bis 60 min. unter Rühren gestrippt. Beim Strippen mit Inertgas werden flüchtige Bestandteile unter Einleiten von Inertgasen in die Flüssigphase bei gleichzeitig angelegtem Vakuum, bei einem absoluten Druck von 5 bis 500 mbar, entfernt. Nach dem Eindosieren von typischerweise 5 bis 20 Gew.-% eines oder mehrerer Alkylenoxide bezogen auf die Menge an vorgelegter H-funktioneller Verbindung, und ggf. von Co-Monomeren, wird der DMC-Katalysator aktiviert.

Die Zugabe eines oder mehrerer Alkylenoxide kann vor, während oder nach dem Aufheizen des Reaktorinhaltes auf Temperaturen von 50 bis 160 °C, bevorzugt 60 bis 140 °C, ganz besonders bevorzugt 70 bis 140 °C geschehen; sie erfolgt bevorzugt nach dem Strippen. Die Aktivierung des Katalysators macht sich durch einen beschleunigten Abfall des Reaktordruckes bemerkbar, wodurch der beginnende Alkylenoxidumsatz / Umsatz des Co-Monomers angezeigt wird.

Dem Reaktionsgemisch kann sodann die gewünschte Menge Alkylenoxid bzw. Alkylenoxidgemisch und ggf. weiteres Co-Monomer kontinuierlich zugeführt werden, wobei eine Reaktionstemperatur von 20 bis 200 °C, bevorzugt aber von 50 bis 160 °C gewählt wird. Die Reaktionstemperatur ist in den vielen Fällen identisch mit der Aktivierungstemperatur.

Oft erfolgt die Katalysatoraktivierung bereits so schnell, dass die Dosierung einer separaten Menge Alkylenoxid / des Co-Monomers zur Katalysatoraktivierung entfallen kann und direkt, gegebenenfalls zunächst mit einer reduzierten Dosierrate, mit der kontinuierlichen Dosierung des Alkylenoxids und ggf. des Co-Monomers begonnen werden kann. Auch kann die Reaktionstemperatur während der Alkylenoxiddosierphase / der Dosierung des Co-Monomers innerhalb der beschriebenen Grenzen variiert werden. Ebenfalls können die Alkylenoxide und das Co-Monomer dem Reaktor auf unterschiedliche Weise zugeführt werden: Möglich ist eine Dosierung in die Gasphase oder direkt in die Flüssigphase, z. B. über ein Tauchrohr oder einen in der Nähe des Reaktorbodens in einer gut durchmischten Zone befindlichen Verteilerring.

Bei DMC-katalysierten Prozessen ist die Dosierung in die Flüssigphase die bevorzugte Variante. Das Alkylenoxid und das Co-Monomer sollten dem Reaktor kontinuierlich derart zugeführt werden, dass die sicherheitstechnischen Druckgrenzen des verwendeten Reaktorsystems nicht überschritten werden. Insbesondere bei der Co-Dosierung von ethylenoxidhaltigen Alkylenoxidgemischen oder reinem Ethylenoxid ist darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden.

Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate und Eduktvorlagen verhindert werden. Wird ein Alkylenoxid- / Co-Monomergemisch dosiert, können die jeweiligen Alkylenoxide und die jeweiligen Co-Monomere dem Reaktor separat oder als Mischung zugeführt werden. Eine Vorvermischung der Alkylenoxide untereinander und mit dem Co-Monomer kann beispielsweise durch ein in der gemeinsamen Dosierstrecke befindliches Mischaggregat erreicht werden ("inline-blending").

Es hat sich auch bewährt, Alkylenoxide und ggf. das Co-Monomer pumpendruckseitig in einen beispielsweise über Wärmetauscher geführten Umpumpkreislauf einzeln oder vorgemischt zu dosieren. Für die gute Durchmischung mit dem Reaktionsmedium ist es dann von Vorteil, ein hochscherendes Mischaggregat in den Alkylenoxid-/ Co-Monomer-/ Reaktionsmediumstrom zu integrieren. Die Temperatur der exothermen ringöffnenden Additionsreaktion wird durch Kühlung auf dem gewünschten Niveau gehalten. Gemäß dem Stand der Technik zur Auslegung von Polymerisationsreaktoren für exotherme Reaktionen (z.B. Ullmann's Encyclopedia of Industrial Chemistry, Vol. B4, pp 167ff, 5th Ed., 1992) erfolgt eine solche Kühlung im Allgemeinen über die Reaktorwand (z.B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten-, Rohrbündel- oder Mischerwärmetauschern. Diese sollten so ausgelegt sein, dass auch zu Beginn der Dosierphase, d. h. bei kleinem Füllstand, effektiv gekühlt werden kann.

Generell sollte in allen Reaktionsphasen durch Auslegung und Einsatz handelsüblicher Rührorgane für eine gute Durchmischung des Reaktorinhaltes gesorgt werden, wobei hier insbesondere ein- oder mehrstufig angeordnete Rührer oder großflächig über die Füllhöhe wirkende Rührertypen geeignet sind (siehe z. B. Handbuch Apparate; Vulkan-Verlag Essen, 1. Aufl. (1990), S.188 - 208). Technisch besonders relevant ist hierbei eine im Mittel über den gesamten Reaktorinhalt eingetragene Mischenergie, die im Allgemeinen im Bereich von 0,2 bis 5 W/1 liegt, mit entsprechend höheren lokalen Leistungseinträgen im Bereich der Rührorgane selbst und ggf. bei niedrigen Füllständen. Um eine

optimale Rührwirkung zu erzielen, können im Reaktor gemäß allgemeinem Stand der Technik Kombinationen aus Stromstörern (z. B. Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Beispielsweise kann es vorteilhaft sein, feststoffhaltige Dispersionen, die zu Reaktionsbeginn beispielsweise bei der Verwendung von Saccharose vorliegen können, besonders intensiv zu durchmischen.

Außerdem sollte insbesondere beim Einsatz fester H-funktioneller Starterverbindungen durch die Wahl des Rühraggregates sichergestellt werden, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restepoxide zusätzlich im Vakuum bei absoluten Drücken im Bereich von 1 bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und gegebenenfalls weitere Stützlager im Behälter auf. Der Antrieb der Rührerwelle kann dabei von oben oder unten erfolgen (mit zentrischer oder exzentrischer Anordnung der Welle).

Alternativ ist es auch möglich, die notwendige Durchmischung ausschließlich über einen Wärmetauscher geführten Umpumpkreislauf zu erzielen oder diesen zusätzlich zum Rühraggregat als weitere Mischkomponente zu betreiben, wobei der Reaktorinhalt nach Bedarf (typischerweise 1 bis 50 mal pro Stunde) umgepumpt wird.

Für die Durchführung des erfindungsgemäßen Verfahrens sind die unterschiedlichsten Reaktortypen geeignet. Vorzugsweise werden zylinderförmige Behälter eingesetzt, welche ein Höhen-/Durchmesserverhältnis von 1:1 bis 10:1 besitzen. Als Reaktorböden kommen beispielsweise Kugel-, Klöpper-, Flach,- oder Konusböden in Frage.

Nach Ende der Dosierung des Alkylenoxids und des Co-Monomers in Schritt a) kann sich eine Nachreaktionsphase anschließen, in der restliches Alkylenoxid und Co-Monomer abreagieren. Das Ende dieser Nachreaktionsphase ist erreicht, wenn kein weiterer Druckabfall im Reaktionskessel feststellbar ist. Spuren unreagierter Alkylenoxide und unreagierten Co-Monomers können nach der Reaktionsphase gegebenenfalls im Vakuum bei einem absoluten Druck von 1 bis 500 mbar oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum (beispielsweise durch Durchleiten von Inertgas bei einem Absolutdruck von 5 bis 500 mbar) entfernt. Das Entfernen flüchtiger Bestandteile, wie beispielsweise nicht umgesetzter Epoxide, entweder im Vakuum oder durch Strippen, erfolgt bei Temperaturen von 20 bis 200 °C, bevorzugt bei 50 bis 160 °C und vorzugsweise unter Rühren. Solche Strippvorgänge können auch in sog. Strippkolonnen durchgeführt werden, in denen dem Produktstrom ein Inertgas- oder Wasserdampfstrom entgegengeleitet wird. Bevorzugt wird das Strippen mit Inertgasen in Abwesenheit von Wasserdampf durchgeführt. Nach Erreichen von Druckkonstanz bzw. nach Entfernen flüchtiger Bestandteile durch Vakuum und/oder Strippen kann das Produkt aus dem Reaktor abgelassen werden.

Wird nicht bereits die gesamte Co-Monomermenge zu Beginn vorgelegt, kann bei Verfahrensvariante A) die Dosierung des Co-Monomers in Schritt a) auch so erfolgen, dass die Alkylenoxiddosierung unterbrochen wird, und nach einer Nachreaktionsphase die Dosierung weiteren Co-Monomers wieder aufgenommen wird. Diese Vorgehensweise kann natürlich während eines Reaktionsansatzes auch mehrmals wiederholt werden. Besonders bevorzugt bei dieser Verfahrensweise ist, dass der abschließende Alkylenoxidblock eine Menge von größer 1 mol Alkylenoxid pro mol aktiver H-Atome aus den als Starterverbindungen eingesetzten H-funktionellen Verbindungen umfasst.

Es ist ebenso möglich, das Verhältnis der Dosiergeschwindigkeiten von Alkylenoxiddosierung und der Dosierung des Co-Monomers während einer parallelen Zudosierdosierung dieser beiden Komponenten gegeneinander kontinuierlich oder stufenweise zu variieren, indem beispielweise das Verhältnis des Dosierstroms des Co-Monomers zu dem des Alkylenoxids / der Alkylenoxide Werte von 0:1 bis 1:0 annimmt.

Ein Charakteristikum von DMC-Katalysatoren ist ihre ausgeprägte Empfindlichkeit gegen hohe Konzentrationen an Hydroxylgruppen, welche beispielsweise durch große Mengen an Startern wie Ethylenglykol, Propylenglykol, Glycerin, Trimethylolpropan, Sorbitol oder Saccharose hervorgerufen werden, und polare Verunreinigungen des Reaktionsgemisches bzw. des Starters oder der Starter. Die DMC-Katalysatoren können dann während der Reaktionsinitiierungsphase nicht in die polymerisationsaktive Form überführt werden. Verunreinigungen können beispielsweise Wasser oder Verbindungen mit einer hohen Zahl in enger Nachbarschaft stehender Hydroxylgruppen wie Kohlenhydrate und Kohlenhydratderivate sein. Auch Substanzen mit in enger Nachbarschaft stehenden Carbonylgruppen bzw. zu Hydroxylgruppen benachbarten Carbonylgruppen wirken sich nachteilig auf die Katalysatoraktivität aus.

Um Starter mit hohen Konzentrationen an OH-Gruppen, bzw. Starter mit als Katalysatorgiften anzusehenden Verunreinigungen dennoch DMC-katalysierten Alkylenoxidadditionsreaktionen unterziehen zu können, sollte die Hydroxylgruppenkonzentration gesenkt bzw. die Katalysatorgifte unschädlich gemacht werden. Hierzu können aus diesen Starterverbindungen mittels basischer Katalyse zunächst Vorpolymerisate hergestellt werden, welche dann nach Aufarbeitung mittels DMC-Katalyse in die erwünschten Alkylenoxidadditionsprodukte hoher Molmasse überführt werden.

Unter diese Vorpolymerisate fallen beispielsweise die oben erwähnten als Starter geeigneten "vorgefertigten Alkylenoxidadditionsprodukte". Nachteilig bei dieser Vorgehensweise ist, dass solche oft mittels basischer Katalyse erhaltenen Vorpolymerisate sehr sorgfältig aufgearbeitet werden müssen, um die Deaktivierung des DMC-Katalysators durch ggf. über die Vorpolymerisate eingeschleppte basische Katalysatorspuren auszuschließen.

Dieser Nachteil kann durch das sogenannte Verfahren der kontinuierlichen Starterdosierung überwunden werden. Hierbei werden kritische Starterverbindungen im Reaktor nicht vorgelegt, sondern neben den Alkylenoxiden dem Reaktor während der Reaktion kontinuierlich zugeführt. Als Startmedium für die Reaktion können in diesem Verfahren Vorpolymerisate vorgelegt werden, auch ist die Verwendung kleiner Mengen des herzustellenden Produktes selbst als Startmedium möglich. Die Notwendigkeit, für weitere Alkylenoxidadditionen geeignete Vorpolymerisate zunächst separat herstellen zu müssen, entfällt somit.

In Variante B) von Schritt a) des erfindungsgemäßen Verfahrens werden daher ein Starterpolyol und der DMC-Katalysator im Reaktorsystem vorgelegt und die H-funktionelle Verbindung kontinuierlich gemeinsam mit dem Alkylenoxid und dem Co-Monomer zugeführt. Als Starterpolyol in Schritt a) sind Alkylenoxidadditionsprodukte wie beispielsweise Polyetherpolyole, Polyesterpolyole, Polyetheresterpolyole, Polycarbonatpolyole, Polyestercarbonatpolyole, Polyethercarbonatpolyole jeweils beispielsweise mit OH-Zahlen im Bereich von 3 bis 1000 mg KOH/g, vorzugsweise von 3 bis 300 mg KOH/g, und / oder gemäß Schritt a) separat hergestellte Hydroxylgruppen tragende Vorstufe, geeignet. Vorzugsweise wird gemäß Schritt a) separat hergestellte Hydroxylgruppen tragende Vorstufe als Starterpolyol in Schritt a) eingesetzt.

In einer weniger bevorzugten Variante dieser Ausführungsform B) ist es ebenfalls möglich, das Verhältnis der Dosiergeschwindigkeiten von Alkylenoxiddosierung und der Dosierung des Co-Monomers während der Zudosierphase der drei Komponenten gegeneinander kontinuierlich oder stufenweise zu varriieren, indem beispielweise das Verhältnis des Dosierstroms des Co-Monomers zu dem des Alkylenoxids / der Epoxide Werte von 0:1 bis 1:0 annimmt. Diese Ausführungsform ist weniger bevorzugt, da nach ihr die Hydroxylgruppen tragende Vorstufe nach Schritt a) in weniger einheitlicher Form erhalten wird.

Vorzugsweise werden in Ausführungsform B) von Schritt a) die Dosierung der H-funktionellen Verbindung und die des Alkylenoxids sowie des Co-Monomers gleichzeitig beendet, oder die H-funktionelle Verbindung und eine erste Teilmenge an Alkylenoxid und eine erste Teilmenge des Co-Monomers werden zunächst gemeinsam zudosiert und anschließend die zweite Teilmenge an Alkylenoxid und Co-Monomer zudosiert, wobei die Summen der ersten und zweiten Teilmenge an Alkylenoxid und der ersten und zweiten Teilmenge an Co-Monomer der Gesamtmenge an in Schritt a) eingesetzter Menge an einem oder mehreren Alkylenoxiden bzw. an einem oder mehreren Co-Monomeren entsprechen. Die erste Teilmenge beträgt vorzugsweise 60 bis 98 Gew. -% und die zweite Teilmenge beträgt 40 bis 2 Gew.-% der insgesamt in Schritt a) zu dosierenden Menge an Alkylenoxid. Die erste Teilmenge beträgt vorzugsweise 0 bis 100 Gew. -% und die zweite Teilmenge beträgt 100 bis 0 Gew.-% der insgesamt in Schritt a) zu dosierenden Menge eines oder mehrerer Co-Monomere.

Wird die Zusammensetzung der Alkylenoxide und / oder die Zusammensetzung / die Dosierrate des einen oder der mehreren Co-Monomere nach Ende der Dosierung der H-funktionellen Verbindung geändert, lassen sich auch nach Verfahrensvariante B) Produkte mit Multiblockstrukturen herstellen. Dabei ist innerhalb der Blöcke eine statistische Verteilung der Co-Monomereinheiten vorgesehen. Auch bei Verfahrensvariante B) ist es bevorzugt, dass die Dosierung des Co-Monomers vor der Alkylenoxiddosierung beendet wird, besonders bevorzugt dergestalt, dass dieser abschließende Alkylenoxidblock eine Menge von größer 1 mol Alkylenoxid pro mol aktiver H-Atome aus den als Starterverbindungen eingesetzten H-funktionellen Verbindungen umfasst. Nach Zudosierung der Reagenzien kann sich eine Nachreaktionsphase anschließen, in der der Verbrauch an Alkylenoxid / Co-Monomer durch Überwachung des Drucks quantifiziert werden kann. Nach Erreichen von Druckkonstanz kann das Endprodukt, gegebenenfalls nach Anlegen von Vakuum oder durch Strippen zur Entfernung von nicht umgesetzen Alkylenoxiden, wie oben beschrieben, abgelassen werden.

In Variante C) von Schritt a) des erfindungsgemäßen Verfahrens können die Hydroxylgruppen tragenden Vorstufen vollkontinuierlich hergestellt werden. Hierzu werden neben Alkylenoxid und der H-funktionellen Verbindung sowie dem Co-Monomer auch der DMC-Katalysator dem Reaktor bzw. einem Reaktorsystem unter Alkoxylierungsbedingungen kontinuierlich zugeführt und das Produkt kontinuierlich dem Reaktor bzw. dem Reaktorsystem nach einer vorwählbaren mittleren Verweilzeit entnommen. Bei Verfahrensvariante C) ist es bevorzugt, dass als Reaktorsystem eine Reaktorkaskade eigesetzt wird, bei der sich zwischen dem Nachreaktor und dem eigentlichen Reaktor ein dritter, kontinuierlich betriebener Reaktor befindet, in den ausschließlich das eine oder mehrere Alkylenoxide kontinuierlich dosiert werden. In einer besonders bevorzugten Ausführungsform der Verfahrensvariante C) umfasst dieser abschließende Alkylenoxidblock eine Menge von größer 1 mol Alkylenoxid pro mol aktiver H-Atome aus den als Starterverbindungen eingesetzten H-funktionellen Verbindungen.

Es können sich kontinuierliche Nachreaktionsschritte, beispielsweise in einer Reaktorkaskade oder in einem Rohrreaktor anschließen. Flüchtige Bestandteile können im Vakuum und/oder durch Strippen, wie oben beschrieben, entfernt werden

Die OH-Zahlen der nach dem DMC-katalysierten Additionsschritt a) erhaltenen Hydroxylgruppen tragenden Vorstufen weisen bevorzugt Werte von 3 mg KOH/g bis 200 mg KOH/g, besonders bevorzugt von 10 bis 60 mg KOH/g, ganz besonders bevorzugt von 20 bis 50 mg KOH/g, auf.

Die OH-Zahl kann z. B. titrimetrisch nach der Vorschrift der DIN 53240 oder spektroskopisch über NIR bestimmt werden.

Unter Äquivalentmolmasse ist die durch die Zahl der aktiven Wasserstoffatome geteilte Gesamtmasse des aktive Wasserstoffatome enthaltenden Materials zu verstehen. Im Falle von Hydroxylgruppen-haltigen Materialien steht sie in folgender Beziehung zur OH-Zahl:

$$\text{Äquivalentmolmasse} = 56100 / \text{OH-Zahl}\,[\text{mgKOH/g}]$$

Den nach Schritt a) des erfindungsgemäßen Verfahrens erhältlichen Hydroxylgruppen tragenden Vorstufen können gegebenenfalls Alterungsschutzmittel wie z. B. Antioxidantien zugesetzt werden.

Der Schritt b) erfolgt bei diesen Verfahrensvarianten wie vorstehend erläutert.

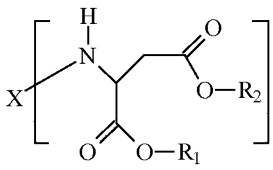

(VIII),.

Das erfindungsgemäße Gewebekleber-System umfasst einen Gewebekleber mit einer Komponente A) sowie einer Komponente B) und/ oder einer Komponente C), wobei die Komponente A) ein isocyanatfunktionelles Präpolymer ist, erhältlich durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, sowie Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie

b) Umsetzung der Hydroxylgrupen tragenden Vorstufe aus Schritt a) mit einem polyfunktionellen Isocyanat zu dem isocyanatfunktionellen Präpolymer,

wobei die Komponente B) ein aminofunktioneller Asparaginsäureester der allgemeinen Formel (I)

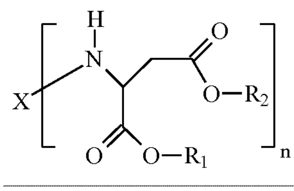

(I)

ist,
wobei

X ein n-wertiger organischer Rest ist,

$R_1$, $R_2$ gleiche oder verschiedene organische Reste sind, die keine Zerewitinoff-aktiven H-Atome aufweisen,

n eine ganze Zahl ≥ 2 ist, wobei der aminofunktionelle Asparaginsäureester ein solcher der allgemeinen Formel (VIII)

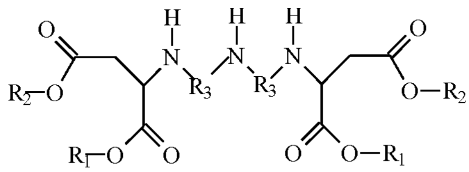

(VIII)

ist,

wobei $R_1$, $R_2$, $R_3$ gleiche oder verschiedene organische Reste sind, die keine Zerewitinoff-aktivenH-Atome aufweisen,

und wobei die Komponente C) ein Umsetzungsprodukt des isocyanatfunktionellen Präpolymers A) mit dem aminofunktionellen Asparaginsäureester B) ist.

Beim Aushärten der Komponenten A) und B) entsteht also ein Polyharnstoffpolymer, wobei die Komponente B) als Härter für die Komponente A) fungiert.

Neben der guten Bioabbaubarkeit der erfindungsgemäßen Gewebekleber-Systeme zeichnen sich diese vor allem durch eine kurze Aushärtezeit aus. So härten die erfindungsgemäßen Gewebekleber-Systeme bei Raumtemperatur und durchschnittlicher Luftfeuchtigkeit auf menschlicher Haut in der Regel binnen weniger als 5 Minuten soweit aus, dass sie an der Oberfläche nicht mehr haftklebrig, also "tacky" sind. Dies kann beispielsweise mit dem Finger überprüft werden. Bevorzugte Gewebekleber-Systeme sind bereits nach weniger als 4 Minuten nicht mehr haftklebrig.

Die Herstellung der aminofunktionellen Asparaginsäureester kann beispielsweise durch (Michael-)Addition eines Dieesters einer difunktionellen ungesättigten organischen Säure wie Diethylmaleat an die primären Aminogruppen eines wenigstens zwei primäre Aminogruppen aufweisenden organischen Amins, wie Bis(hexamethylen)-triamin, erfolgen. Die Herstellung ist beispielsweise aus EP 11153810.4 bekannt, deren Inhalt hiermit vollständig in die vorliegende Offenbarung aufgenommen wird. Neben den Diestern der Maleinsäure kommen beispielsweise auch die Diester der Tetrahydrophthalsäure, insbesondere solche der 3,4,5,6-Tetrahydrophthalsäure sowie Kombinationen hiervon in Frage.

Selbstverständlich können in die Gewebekleber-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe eingearbeitet werden.

Die Wirkstoffe können als reiner Wirkstoff oder aber in verkapselter Form vorliegen, um beispielsweise eine zeitlich verzögerte Abgabe zu erzielen. Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxidfreisetzende Komponente, bevorzugt L-Arginin oder eine L-Argininhaltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF $\alpha$; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere $\beta$-Carotin, Vitamin E, Gruppe der Tocopherole, insbesondere α Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet, die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere

Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen der erfindungsgemäßen Zusammensetzung.

Bei dem erfindungsgemäßen Gewebekleber-System ist der aminofunktionelle Asparaginsäureester ausgewählt aus Strukturen der allgemeinen Formel (VIII)

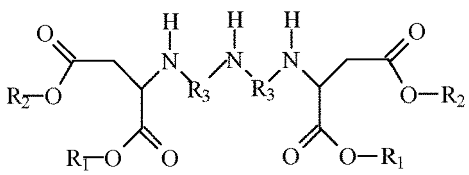

~~(IX)~~ (VIII)

wobei R1, R2, R3 gleiche oder verschiedene organische Reste sind, die keine Zerewitinoff aktiven H-Atome aufweisen, wobei R1 und R2 insbesondere ausgewählt sind aus Methyl-, Ethyl-, Propyl- und Butyl-Resten, sowie R3 insbesondere ausgewählt ist aus geradkettigen oder verzweigten Alkylendiradikalen mit 1 bis 12 Kohlenstoffatomen, vorzugsweise mit 3 bis 7 Kohlenstoffatomen.

Das erfindungsgemäße Gewebekleber-System ist jedoch nicht auf den alleinigen Einsatz der vorgenannten Härter (Komponente B) beschränkt, sondern kann auch noch einen oder mehrere zusätzliche Härter aufweisen. So umfasst das erfindungsgemäße Gewebekleber System nach einer besonders bevorzugten Ausführungsform ferner einen weiteren Härter, der insbesondere aus Polyolen mit einer zahlenmittleren Molmasse von 1000 Da oder weniger ausgewählt ist, insbesondere von 600 Da oder weniger, weiter bevorzugt 400 Da oder weniger oder gar 300 Da oder weniger. Als Polyole eignen sich beispielsweise PEG oder PPG. Durch den Zusatz dieser zusätzlichen Härter kann die Aushärtungsgeschwindigkeit des erfindungsgemäßen Gewebekleber-Systems beeinflusst, das heißt in der Regel verkürzt werden, so dass eine bedarfsgerechte Konfektionierung des Gewebeklebers erfolgen kann.

Nach einer weiteren Ausgestaltung des erfindungsgemäßen Gewebekleber-Systems umfasst dieses ferner eine flächige Schutzschicht, mit welcher der Gewebekleber in Kontakt gebracht werden kann, wobei die Schutzschicht insbesondere eine Metallfolie, eine Kunststoffolie, ein Vlies, ein Gewebe, ein Gelege, ein Gewirke, oder eine Kombination hiervon ist. Auf diese Weise kann der Kleber auf eine zu verklebende Stelle, wie beispielsweise eine Wunde, aufgedrückt werden um die Adhäsion weiter zu verbessern und ein Verlaufen des Klebstoffs zu verhindern, ohne dass der Anwender dabei mit dem Klebstoff in Kontakt kommt. Zudem kann auf diese Weise eine Blutung leichter gestoppt werden, da durch den bei der Applikation aufgebrachten Anpressdruck auch eine lokale Kompression der Blutgefäße erfolgt. Die Schutzschicht kann anschließend entweder an Ort und Stelle verbleiben, oder aber auch abgelöst werden. Hierzu ist die Schutzschicht zweckmäßigerweise zumindest auf ihrer dem Klebstoff zugewandten Seite mit einer antiadhäsiv-Beschichtung versehen, wie beispielsweise einer Silikonisierung der betreffenden Fläche.

Die Erfindung betrifft des Weiteren ein Dosiersystem mit wenigstens zwei Kammern für ein Gewebekleber-System gemäß dieser Erfindung, wobei das Dosiersystem dadurch gekennzeichnet ist, dass in der einen Kammer die Komponente A) und in der anderen Kammer die Komponente B) sowie gegebenenfalls Komponente C) des Gewebekleber-Systems enthalten sind.

Ein weiterer Gegenstand der Erfindung sind die aus dem erfindungsgemäßen Gewebekleber-System erhältlichen Klebfilme sowie daraus hergestellte Verbundteile.

Gegenstand der vorliegenden Erfindung ist zuletzt auch ein Verfahren zum Verschließen oder Verbinden von Zellgeweben bei dem das erfindungsgemäße Gewebekleber-System verwendet wird.

Die vorliegende Erfindung wird im Folgenden anhand von Ausführungsbeispielen näher erläutert. Sofern nicht abweichend angegeben beziehen sich alle Prozentangaben auf das Gewicht.

## Methoden:

Die Bestimmung der OH-Zahlen erfolgte gemäß der Vorschrift der DIN 53240.

Die Viskositäten der Polyole wurden mittels Rotationsviskosimeter (Physica MCR 51, Hersteller: Anton Paar) nach der Vorschrift der DIN 53018 ermittelt.

Das Zahlenmittel $M_n$ und das Gewichtsmittel $M_w$ des Molekulargewichts sowie die Polydispersität ($M_w/M_n$) wurden mittels Gelpermeations-Chromatographie (GPC) bestimmt. Es wurde vorgegangen nach DIN 55672-1: "Gelpermeationschromatographie, Teil 1 - Tetrahydrofuran als Elutionsmittel" (SECurity GPC-System von PSS Polymer Service, Flussrate 1.0 ml/min; Säulen: 2×PSS SDV linear M, 8x300 mm, 5 μm; RID-Detektor). Dabei wurden Polystyrolproben bekannter Molmasse zur Kalibrierung verwendet.

NCO-Gehalte wurden, wenn nicht ausdrücklich anders erwähnt, volumetrisch gemäß DIN-EN ISO 11909 bestimmt.

Die Bestimmung des Restmonomergehalts erfolgte nach DIN ISO 17025.

## Herstellung der Polyole

Hydroxylgruppen tragende Vorstufe 1 (Polyol 1):

In einem 2 Liter-Edelstahldruckreaktor werden 98,1 g eines auf Glycerin gestarteten Poly(oxypropylen)triols mit OH-Zahl = 400 mg KOH/g, 48,4 g Dilactid sowie 0,107 g DMC-Katalysator (hergestellt gemäß WO 01/80994 A1, dortiges Beispiel 6) unter Stickstoff vorgelegt und dann auf 100°C aufgeheizt. Nach 30 min Strippen mit Stickstoff bei 0,1 bara wird die Temperatur auf 130°C erhöht und bei dieser Temperatur dann eine Mischung aus 701,8 g Ethylenoxid und 217,8 g Propylenoxid innerhalb von 130 min dosiert. Nach einer Nachreaktionszeit von 45 min bei 130°C werden leichtflüchtige Anteile bei 90°C für 30 min im Vakuum abdestilliert und das Reaktionsgemisch anschließend auf Raumtemperatur abgekühlt.

Produkteigenschaften:

| | |
|---|---|
| OH-Zahl: | 33,7 mg KOH/g |
| Viskosität (25°C): | 1370 mPas |
| Polydispersität (Mw/Mn): | 1,13 |

Hydroxylgruppen tragende Vorstufe 2 (Polyol 2):

In einem 2 Liter-Edelstahldruckreaktor werden 140,0 g eines auf Propylenglykol gestarteten Poly(oxypropylen)diols mit OH-Zahl = 260 mg KOH/g, 145,3 g Dilactid sowie 0,087 g DMC-Katalysator (hergestellt gemäß WO 01/80994 A1, dortiges Beispiel 6) unter Stickstoff vorgelegt und dann auf 100°C aufgeheizt. Nach 15 min Strippen mit Stickstoff bei 0,1 bara wird die Temperatur auf 130°C erhöht und bei dieser Temperatur dann eine Mischung aus 526,8 g Ethylenoxid und 54,5 g Propylenoxid innerhalb von 80 min dosiert. Nach einer Nachreaktionszeit von 75 min bei 130°C werden leichtflüchtige Anteile bei 90°C für 30 min im Vakuum abdestilliert und das Reaktionsgemisch anschließend auf Raumtemperatur abgekühlt.

Produkteigenschaften:

| | |
|---|---|
| OH-Zahl: | 29,3 mg KOH/g |
| Viskosität (25°C): | 1185 mPas |
| Polydispersität (Mw/Mn): | 1,41 |

**Herstellung der isocyanatfunktionellen Präpolymere:**

Synthese des isocyanatfunktionellen Präpolymers 1:

183,1 g Hexamethylendiisocyanat (HDI) und 0,9 g Benzoylchlorid wurden in einem 1 l Vierhalskolben vorgelegt. Innerhalb von 2h wurden bei 80°C 478,7 g des Polyols 2 hinzugefügt und 1 h nachgerührt. Anschließend wurde durch Dünnschichtdestillation bei 130°C und 0,13 mbar das überschüssige HDI abdestilliert. Man erhielt das Präpolymer 1 mit einem NCO-Gehalt von 2,38%. Der Restmonomerengehalt betrug < 0,03 % HDI. Viskosität: 4930 mPas/23°C

Synthese des isocyanatfunktionellen Präpolymers 2:

293 g HDI und 1,5 g Benzoylchlorid wurden in einem 1 l Vierhalskolben vorgelegt. Innerhalb von 2h wurden bei 80°C 665,9 g des Polyols 1 hinzugefügt und 1 h nachgerührt. Anschließend wurde durch Dünnschichtdestillation bei 130°C und 0,13 mbar das überschüssige HDI abdestilliert. Man erhielt das Präpolymer 2 mit einem NCO-Gehalt von 2,37%. Der Restmonomerengehalt betrug < 0,03 % HDI. Viskosität: 5740 mPas/23°C

**Herstellung der Aspartat-Härter:**

Aspartat A:

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Es hatte eine quantitative Umsetzung stattgefunden.

Aspartat B:

Zu 2 Mol Diethylmaleat wurde unter Stickstoffatmosphäre langsam 1 Mol Bis(hexamethylen)-triamin getropft, so dass die Reaktionstemperatur 60°C nicht überschritt. Anschließend wurde so lange auf 60°C erwärmt, bis kein Diethylmaleat mehr im Reaktionsgemisch nachweisbar war. Es hatte eine quantitative Umsetzung stattgefunden.

**Herstellung der Gewebekleber:**

Herstellung des Gewebeklebers 1:

4 g des isocyanatfunktionellen Präpolymers 2 wurden mit einer äquivalenten Menge des Aspartats A in einem Becher gut verrührt. Die Reaktionsmischung wurde unmittelbar danach auf das zu klebende Gewebe dünn aufgetragen. Eine Aushärtung zu einem durchsichtigen Film mit einer damit verbundenen starken Klebung hatte innerhalb von 2 min stattgefunden. Die Oberfläche des Klebstoffes war nach 6 min nicht mehr klebrig. Die Verarbeitungszeit hat 5 min 45 s betragen.

Herstellung des Gewebeklebers 2:

4 g des isocyanatfunktionellen Präpolymers 1 wurden mit einer äquivalenten Menge des Aspartats B in einem Becher gut verrührt. Die Reaktionsmischung wurde unmittelbar danach auf das zu klebende Gewebe dünn aufgetragen. Eine Aushärtung zu einem durchsichtigen Film mit einer damit verbundenen starken Klebung hatte innerhalb von 1 min stattgefunden. Die Oberfläche des Klebstoffes war nach 3 min nicht mehr klebrig. Die Verarbeitungszeit hat 1 min 30 s betragen.

**Bestimmung der Bioabbaubarkeit:**

Das zu prüfende Gewebekleber-System wurde in einem Rohr (Durchmesser 0,5 cm, Länge 2 cm) zur Aushärtung gebracht. Der dabei entstandene 2.7 g schwere Prüfkörper wurde in 10 ml Pufferlösung (pH 7,4, Aldrich P-5368) so lange bei 60°C bzw. 37°C im Schüttelinkubator mit 150 U/min geschüttelt, bis sich das Material vollständig, d.h. ohne Bodensatz aufgelöst hatte.

Die Proben waren nach den folgenden Zeiträumen vollständig abgebaut:

Gewebekleber 1:11 Wochen bei 60°C

Gewebekleber 2: 6 Wochen bei 60°C

**Bestimmung der Cytotoxizität:**

Der ausgehärtete Gewebekleber 2 wurde gemäß ISO 10993-5:2009 mit L 929 Zellen auf Zytotoxizität geprüft. Das Material hat sich als nicht zytotoxisch erwiesen.

**Beschleunigung der Aushärtungsgeschwindigkeit:**

Um die Aushärtungsgeschwindigkeit des Gewebeklebers 1 zu erhöhen, und das System in einem 4:1-Doppelkammerspritzensystem applizieren zu können, wurde zu dem Härter Aspartat A so viel Polyethylenglycol (PEG) 200 zugemischt, dass ein Mischungsverhältnis von 4 ml isocyanatfunktionellem Präpolymer 2 und 1 ml Härter entstand. Die Aushärtungszeit verkürzte sich auf 1 min 30 s, was der Verarbeitungszeit entsprach.

Um die Aushärtungsgeschwindigkeit weiter zu erhöhen, was bei der Behandlung starker Blutungen oder Leckagen essentiell ist, wurden unterschiedliche Mischungen aus PEG 200, sowie den Aspartaten A und B hergestellt. Die Mengen wurden dabei so gewählt, dass das Volumenverhältnis von Präpolymer zu Härter 4:1 blieb.

| Härter | Mischungsverhältnis | Verarbeitungszeit |
|---|---|---|
| Aspartat A | / | 5 min 45 s |
| Aspartat A/PEG 200 | 0,57/0,43 | 1 min 40 s |
| Aspartat A/Aspartat B/PEG 200 | 0,369/0,098/0,53 | 1 min 10 s |
| Aspartat A/Aspartat B/PEG 200 | 0,29/0,175/0,55 | 40 s |

**In vivo-Versuche am Hausschwein mit einer Mischung aus isocyanatfunktionellem Präpolymer 2 und Aspartat A/Aspartat B/PEG 200 im Verhältnis 0,29/0,175/0,55:**

Behandlung einer Lungenfistel:

Ein ca. 4 cm großes Stück der Lunge wurde reseziert, wobei eine Lungenfistel entstand. Der Durchmesser des Bronchus betrug ca. 3 mm. Es kam zusätzlich zu einer arteriellen Blutung. Ca. 3 ml des Klebstoffs wurden aus einer 4:1 Doppelkammerspritze der Firma Medmix aufgetragen. Der Klebstoff wurde mit einer geeigneten Folie auf die Wunde gedrückt, um ein Verlaufen zu verhindern. Der Klebstoff härtete innerhalb von ca. 30 s aus. Die Lunge war abgedichtet, die Blutung gestillt. Der Klebstoff hat einem Beatmungsdruck von 22 mm Hg standgehalten.

Vergleichsversuch Lungenfistel- Abdichtung mit Fibrinkleber:

Die gleiche Prozedur wurde wiederholt. Statt des beschriebenen Klebstoffes wurde Fibrin (Tisseel) eingesetzt. Die Vorbereitungszeit zur Mischung des Fibrinklebers betrug ca. 10 min. Nach Auftragen auf die Fistel kam es instantan zur Ausbildung eines Blutpfropfes, unter dem jedoch weiterhin Luft ausgetreten ist. Die Fistel konnte nicht abgedichtet werden.

Stichverletzung Herz:

Mit einem Skalpell wurde am Herz die Koronarvene im linken Ventrikel verletzt, wobei eine ca. 1 cm lange Wunde mit einer spritzenden Blutung erzeugt wurde. 5 ml des Klebstoffs wurden aus einer 4:1 Doppelkammerspritze der Firma Medmix aufgetragen. Der Klebstoff wurde mit einer geeigneten Folie auf die Wunde gedrückt, um ein Verlaufen zu verhindern. Die Wunde konnte innerhalb von 40 s komplett abgedichtet werden und hielt einem Blutdruck von 140 mm Hg stand.

## Patentansprüche

1. Gewebekleber-System, umfassend einen Gewebekleber mit einer Komponente A) sowie einer Komponente B) und/oder einer Komponente C), wobei
die Komponente A) ein isocyanatfunktionelles Präpolymer ist, erhältlich durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, sowie Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie
b) Umsetzung der Hydroxylgruppen tragenden Vorstufe aus Schritt a) mit einem polyfunktionellen Isocyanat zu dem isocyanatfunktionellen Präpolymer,

wobei die Komponente B) ein aminofunktioneller Asparaginsäureester der allgemeinen Formel (I)

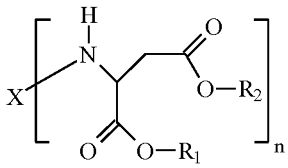
(I)

ist,
wobei

X ein n-wertiger organischer Rest ist,
$R_1$, $R_2$ gleiche oder verschiedene organische Reste sind, die keine Zerewitinoff-aktiven H-Atome aufweisen,
n eine ganze Zahl ≥ 2 ist, wobei der aminofunktionelle Asparaginsäureester ein solcher der allgemeinen Formel (VIII)

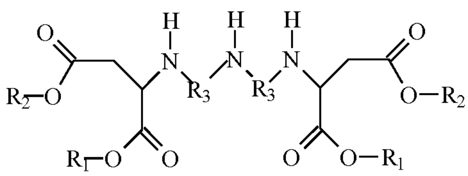
(VIII)

ist,

wobei $R_1$, $R_2$, $R_3$ gleiche oder verschiedene organische Reste sind, die keine Zerewitinoff-aktiven H-Atome aufweisen,
und wobei die Komponente C) ein Umsetzungsprodukt des isocyanatfunktionellen Präpolymers A) mit dem aminofunktionellen Asparaginsäureester B) ist.

2. Gewebekleber-System nach Anspruch 1, **dadurch gekennzeichnet, dass** die H-funktionelle Starterverbindung 1 bis 35 Zerewitinoff-aktive H-Atome aufweist.

3. Gewebekleber-System nach Anspruch 1 oder 2, **dadurch gekennzeichnet, dass** die H-funktionelle Starterverbindung ein mittleres Molgewicht von 17 bis 10000 g/mol aufweist.

4. Gewebekleber-System nach einem der vorstehenden Ansprüche, **dadurch gekennzeichnet, dass** die Alkylenoxidverbindung aus solchen mit 2 bis 24 Kohlenstoffatomen ausgewählt ist.

5. Gewebekleber-System nach Anspruch 4, **dadurch gekennzeichnet, dass** die Alkylenoxidverbindung aus Ethylenoxid und/ oder Propylenoxid ausgewählt ist, wobei der Anteil an Ethylenoxideinheiten in der Polymerkette der Hydroxylgruppen tragenden Vorstufe wenigstens 40 Gew.-% beträgt.

6. Gewebekleber-System nach einem der vorstehenden Ansprüche, **dadurch gekennzeichnet, dass** das bei der Herstellung der Hydroxylgruppen tragenden Vorstufe gewählte Stoffmengenverhältnis von Alkylenoxidverbindung zu Co-Monomer 200 : 1 bis 1 : 1 beträgt.

**7.** Gewebekleber-System nach einem der vorstehenden Ansprüche, **dadurch gekennzeichnet, dass** das polyfunktionelle Isocyanat ausgewählt ist aus aliphatischen Isocyanaten.

**8.** Gewebekleber-System nach einem der vorstehenden Ansprüche, **dadurch gekennzeichnet, dass** das Gewebekleber-System ferner einen weiteren Härter umfasst.

**9.** Gewebekleber-System nach einem der vorstehenden Ansprüche, **dadurch gekennzeichnet, dass** das Gewebekleber-System ferner eine flächige Schutzschicht umfasst, mit welcher der Gewebekleber in Kontakt gebracht werden kann.

**10.** Dosiersystem mit wenigstens zwei Kammern für ein Gewebekleber-System nach einem der Ansprüche 1 bis 9, **dadurch gekennzeichnet, dass** in der einen Kammer die Komponente A) und in der anderen Kammer die Komponente B) des Gewebekleber-Systems enthalten sind.

## Claims

**1.** A tissue adhesive system comprising a tissue adhesive comprising a component A), a component B) and/or a component C),
wherein component A) is an isocyanate functional prepolymer obtained by

a) reacting an H-functional starter compound having at least one Zerewitinoff active H atom with an alkylene oxide and a comonomer to a preliminary stage bearing hydroxyl groups, wherein said comonomer is selected from the group comprising lactides, glycolides and mixtures thereof and combinations of lactides and/or glycolides with cyclical dicarboxylic acid anhydrides and wherein said comonomer is integrated through statistical copolymerization into the polymer chain(s) of said preliminary stage bearing hydroxyl groups, and
b) reacting the preliminary stage bearing hydroxyl groups from step a) with a polyfunctional isocyanate to said isocyanate functional prepolymer,

wherein component B) is an amino functional aspartic acid ester of a general formula (I)

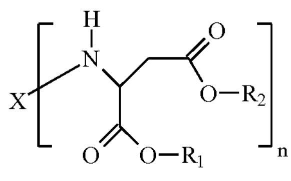
(I)

wherein

x is an n-value organic radical,
$R_1$, $R_2$ have equal or different organic radicals having no Zerewitinoff active H atoms,
n is a whole number ≥ 2, wherein the amino functional aspartic acid ester is of a general formula (VIII)

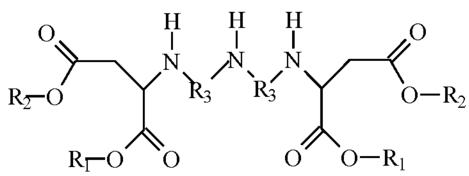
(VIII),

wherein $R_1$, $R_2$, $R_3$ have equal or different organic radicals having no Zerewitinoff active H atoms, and wherein component C) is a reaction product of said isocyanate functional prepolymer A) with said amino functional aspartic acid esters B).

**2.** Tissue adhesive system according to claim 1, **characterized in that** said H-functional starter compound has 1 to

35 Zerewitinoff active H atoms.

**3.** Tissue adhesive system according to claim 1 or 2, **characterized in that** said H-functional starter compound has an average molar weight of 17 to 10000 g/mol.

**4.** Tissue adhesive system according to any of the preceding claims, **characterized in that** said alkylene oxide compound is selected from alkylene oxide compounds comprising 2 to 24 carbon atoms.

**5.** Tissue adhesive system according to claim 4, **characterized in that** said alkylene oxide compound is selected from ethylene oxide and/or propylene oxide, wherein the share of ethylene oxide units in said polymer chain of said preliminary stage bearing hydroxyl groups is at least 40% by weight.

**6.** Tissue adhesive system according to any of the preceding claims, **characterized in that** the molar ratio of said alkylene oxide compound to a comonomer selected during the production of said preliminary stage bearing hydroxyl groups is 200:1 to 1:1.

**7.** Tissue adhesive system according to any of the preceeding claims, **characterized in that** the polyfunctional isocyanate is selected from aliphatic isocyanates.

**8.** Tissue adhesive system according to any of the preceding claims, **characterized in that** said tissue adhesive system comprises an additional hardener.

**9.** Tissue adhesive system according to any of the preceding claims, **characterized in that** said tissue adhesive system comprises a flat protective layer with which the tissue adhesive can be brought into contact.

**10.** A dispensing system comprising at least two chambers for the tissue adhesive system according to any of claims 1 to 9, **characterized in that** said component A) is contained in a one chamber and said component B) of the tissue adhesive system is contained in another chamber.

## Revendications

**1.** Système de colle pour tissus, comprenant une colle pour tissus avec un composant A) ainsi qu'un composant B) et/ou un composant C),
où le composant A) est un prépolymère à fonction isocyanate, disponible grâce à

a) la réaction d'un composé de départ fonctionnel H d'au moins un atome d'hydrogène actif de Zerewitinoff avec un composé d'oxyde d'alkylène et un co-monomère pour donner un précurseur portant des groupes hydroxyles,
où le co-monomère est choisi à partir du groupe comprenant des lactides, des glycolides ainsi que des combinaisons de ces composés et des combinaisons de lactides et/ou de glycolides avec des anhydrides d'acides dicarboxyliques cycliques et
où le co-monomère est intégré à la ou aux chaîne(s) polymères du précurseur portant des groupes hydroxyles au moyen d'une copolymérisation statistique, ainsi que
b) la réaction d'un précurseur portant des groupes hydroxyles de l'étape a) avec un isocyanate polyfonctionnel pour donner un prépolymère à fonction isocyanate,

où le composant B) est un ester d'acide aspartique aminofonctionnel de la formule générale (I)

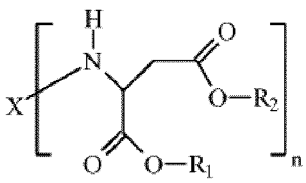
(I)

où

X est un radical organique d'une valence n,
$R_1$, $R_2$ sont des radicaux identiques ou différents ne présentant aucun atome d'hydrogène actif de Zerewitinoff,
n est un nombre entier ≥ 2,
où l'ester d'acide aspartique aminofonctionnel est du type de la formule générale (VIII)

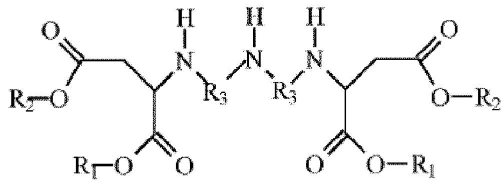

(VIII)

où $R_1$, $R_2$, $R_3$ sont des radicaux identiques ou différents ne présentant aucun atome d'hydrogène actif de Zerewitinoff,
et où le composant C) est un produit de la réaction du prépolymère à fonction isocyanate A) avec l'ester d'acide aspartique aminofonctionnel B).

2. Système de colle pour tissus selon la revendication 1, **caractérisé en ce que** le composé de départ fonctionnel H présente entre 1 et 35 atomes d'hydrogène actifs de Zerewitinoff.

3. Système de colle pour tissus selon la revendication 1 ou 2, **caractérisé en ce que** le composé de départ fonctionnel H présente un poids moléculaire moyen de 17 à 10000 g/mol.

4. Système de colle pour tissus selon l'une des revendications susmentionnées, **caractérisé en ce que** le composé d'oxyde d'alkylène est choisi à partir de ceux-ci avec 2 à 24 atomes de carbone.

5. Système de colle pour tissus selon la revendication 4, **caractérisé en ce que** le composé d'oxyde d'alkylène est choisi à partir de l'oxyde d'éthylène et/ou de l'oxyde de propylène, où la part d'unités d'oxyde d'éthylène dans la chaîne polymère du précurseur portant des groupes hydroxyles s'élève au moins à 40 % du poids.

6. Système de colle pour tissus selon l'une des revendications susmentionnées, **caractérisé en ce que** le rapport molaire du composé d'oxyde d'alkylène choisi lors de la préparation du précurseur portant des groupes hydroxyles au co-monomère est de 200: 1 à 1: 1.

7. Système de colle pour tissus selon l'une des revendications susmentionnées, **caractérisé en ce que** l'isocyanate polyfonctionnel est choisi à partir d'isocyanates aliphatiques.

8. Système de colle pour tissus selon l'une des revendications susmentionnées, **caractérisé en ce que** le système de colle pour tissus comprend aussi un autre durcisseur.

9. Système de colle pour tissus selon l'une des revendications susmentionnées, **caractérisé en ce que** le système de colle pour tissus comprend aussi une couche protectrice en surface avec laquelle la colle pour tissus peut entrer en contact.

10. Système de dosage avec au moins deux chambres pour un système de colle pour tissus selon les revendications 1 à 9, **caractérisé en ce que** qu'une chambre contient le composant A) et que une autre chambre contiennent le composant B) du système de colle pour tissus.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2011808 A1
- EP 2145634 A1
- WO 2009106245 A2
- EP 2336212 A1
- US 2003032734 A1
- US 20050129733 A1
- EP 1525244 A1
- EP 2046861 A
- US 3404109 A1
- US 3829505 A1
- US 3941849 A1
- US 5158922 A1
- US 5470813 A1
- EP 700949 A1
- EP 743093 A1
- EP 761708 A1
- WO 9740086 A1
- WO 9816310 A1
- WO 0047649 A1
- US A5158922 A1
- JP 4145123 A
- WO 0139883 A1
- WO 0180994 A1
- US 4987271 A1
- DE 3132258 A1
- EP 406440 A1
- US 5391722 A1
- US 5099075 A1
- US 4721818 A1
- US 4877906 A1
- EP 385619 A1
- EP 11153810 A


### In der Beschreibung aufgeführte Nicht-Patentliteratur


- *Ullmann's Encyclopedia of Industrial Chemistry,* 1992, vol. B4, 167ff
- Handbuch Apparate. Vulkan-Verlag Essen, 1990, 188-208